(19) Europäisches Patentamt
European Patent Office
Office européen des brevets

(11) Veröffentlichungsnummer: **0 203 379**
**A1**

# (12) EUROPÄISCHE PATENTANMELDUNG

(21) Anmeldenummer: **86105676.0**

(22) Anmeldetag: **24.04.86**

(51) Int. Cl.⁴: **C 07 C 83/10**

(30) Priorität: **26.04.85 YU 709/85**

(43) Veröffentlichungstag der Anmeldung: **03.12.86 Patentblatt 86/49**

(84) Benannte Vertragsstaaten: **AT CH DE LI NL**

(71) Anmelder: **LEK, tovarna farmacevtskih in kemicnih izdelkov, n.sol.o, Verovskova 57, 61000 Ljubljana (YU)**

(72) Erfinder: **Boznar, Breda, Dipl.-Ing., Poljanska 34, YU-64220 Skofja Loka (YU)**
Erfinder: **Zmitek, Janko, Mag. Dipl.-Ing., Pokiukarjeva 6, YU-61000 Ljubljana (YU)**
Erfinder: **Kocjan, Darko, Dr. Ing., Ul. narodne zascite 1, YU-61000 Ljubljana (YU)**

(74) Vertreter: **Patentanwälte Müller-Boré, Deufel, Schön, Hertel, Lewald, Otto, Postfach 26 02 47 Isartorplatz 6, D-8000 München 26 (DE)**

## (54) Verfahren zur Herstellung von 2-(4-(Isobutylphenyl)-propiohydroxamsäure.

(57) Es wird ein neues, technologisch leicht durchführbares Verfahren zur Herstellung von 2-(4-Isobutylphenyl)-propiohydroxamsäure beschrieben, bei welchem man zwei Moleküle 2-(4-Isobutylphenyl)-propionsäure in Anwesenheit von N, N'-dicyclohexyl-carbodiimid bei Raum- oder leicht erhöhter Temperatur in einem inerten organischen Lösungsmittel, z.B. Methylenchlorid, Acetonitril oder Chloroform, zu Bis-/2-(4-Isobutylphenyl)-propionsäure/-anhydrid dehydriert, mit welchem dann in einer Methylenchlorid-Lösung bei Raum- oder leicht erhöhter Temperatur Hydroxylamin zur gewünschten Verbindung acyliert wird. Die Umsetzungen werden unter Ausschluß von Luftfeuchtigkeit durchgeführt. 2-(4-Isobutylphenyl)-propiohydroxamsäure (Freiname Ibuproxam) besitzt bemerkenswerte analgetische, antipyretische und antiinflammatorische Wirkungen.

EP 0 203 379 A1

ACTORUM AG

LEK, tovarna farmacevtskih in kemicnih izdelkov, n.sol.o.
Bt - L 1259 EU

Verfahren zur Herstellung von 2-(4-(Isobutylphenyl)-propiohydroxamsäure

Gebiet der Technik, in welches die Erfindung eingeordnet wird

(Internationale Patentklassifikation C 07 C 83/10)

Die vorliegende Erfindung betrifft ein neues Verfahren zur Herstellung von 2-(4-Isobutylphenyl)-propiohydroxamsäure der Formel

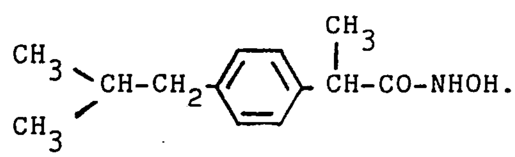

Die Verbindung ist unter dem Freinamen Ibuproxam bekannt und besitzt sehr ausgeprägte analgetische, antipyretische und antiinflammatorische Eigenschaften.

Technisches Problem

Es besteht ein ständiges Bedürfnis nach neuen, technologisch leicht durchführbaren Verfahren, nach welchen die gewünschte Verbindung Ibuproxam auf eine einfache und schnelle Weise mit guter Ausbeute und grosser Reinheit herstellbar wäre.

Stand der Technik

2-(4-Isobutylphenyl)-propiohydroxamsäure wurde zuerst in DE-PS 2 400 531 als eine neue Verbindung, die eine bedeutende analgetische, antipyretische und antiinflammatorische Wirkung aufweist, beschrieben.

In der erwähnten Patentschrift wurde auch ein Verfahren zur Herstellung von Ibuproxam beschrieben, bei welchem eine methanolische Hydroxylamin-Lösung nach bekannten Methoden mit 2-(4-Isobutylphenyl)-propionsäure-äthylester acyliert wird. Nach einer Variante erfolgt die Umsetzung bei Rückflusstemperatur des Reaktionsgemisches. Die Umsetzung erfolgt zwar rasch, die Ausbeute an der gewünschten Verbindung ist jedoch niedrig (etwa 50 %). Nach einer anderen Variante, die unter Kühlung des Reaktionsgemisches verläuft, ist die Ausbeute zwar höher (etwa 73 %), die Umsetzung verläuft jedoch langsam.

Die methanolische Hydroxylamin-Lösung kann auf bekannte Weise aus dem Hydroxylamin-Hydrochlorid-Salz hergestellt werden. Auch das 2-(4-Isobutylphenyl)-propionsäure-äthylester kann auf bekannte Weise durch Veresterung der 2-(4-Isobutylphenyl)-propionsäure, die eine bekannte und in der Literatur beschriebene Verbindung ist, mit Aethanol in Anwesenheit der Schwefelsäure bei Rückflusstemperatur des Reaktionsgemisches in einer etwa 80 %igen Ausbeute hergestellt werden.

Reaktionsschema:

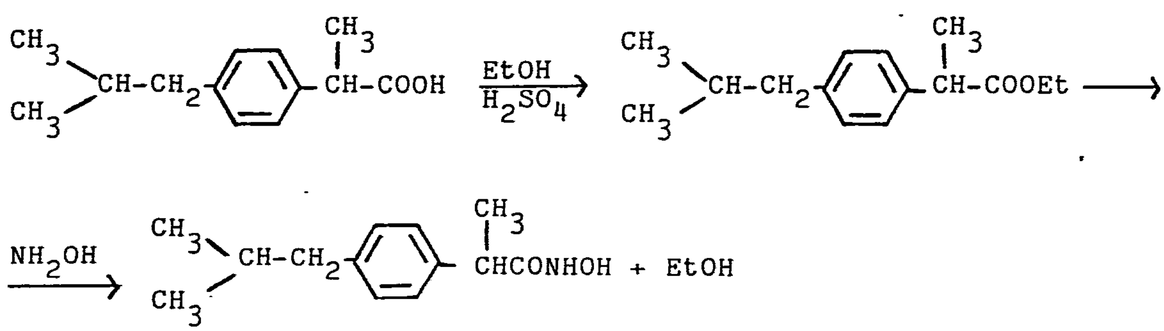


Die Gesamtausbeute der Umsetzung beträgt je nach der gewählten Temperatur 40 bzw. 59 %.

Ein Nachteil des angegebenen Verfahrens zur Herstellung von Ibuproxam sind niedrige Gesamtausbeuten bzw. der langsame Verlauf der Umsetzung.

Aus der Literatur sind Verfahren zur Herstellung von Hydroxamsäuren durch Acylierung von Hydroxylamin mit verschiedenen Acylierungsmitteln, z.B. Carbonsäure-chloriden oder -äthylestern, bekannt. Für die Acylierung mit Carbonsäure-äthylestern, die in der DE-PS 2 400 531 verwendet wurde, wird angeführt, dass sie selektiver ist und deswegen auch am häufigsten verwendet wird. Die Acylierung von Hydroxylamin mit Carbonsäure-anhydriden ist in der Literatur nicht eingehend beschrieben. In Houben-Weyl, Methoden der organischen Chemie, Sauerstoffverbindungen III, Band VIII, 688 (1952), wird die Acylierung von Hydroxylamin mit überschüssigen aliphatischen Säureanhydriden beschrieben, wobei es zur O-Acylierung von Hydroxylamin kommt.

Beschreibung der Lösung des technischen Problems mit Ausführungsbeispielen

Der Erfindung liegt die Aufgabe zugrunde, ein Verfahren zu schaffen, bei welchem die 2-(4-Isobutylphenyl)-propiohydroxamsäure mit einer grossen Gesamtausbeute innerhalb kurzer Reaktionszeiten, bei Zimmer- oder leicht erhöhter Temperatur hergestellt werden könnte, wobei das gewünschte Produkt auf einfache Weise und mit grosser Reinheit isoliert werden könnte.

Diese Aufgabe wird durch die Acylierung von Hydroxylamin mit Bis/2-(4-isobutylphenyl)-propionsäure/-anhydrid gelöst.

Die Umsetzung findet bei Zimmer- oder leicht erhöhter Temperatur in Anwesenheit von inerten organischen Lösungsmitteln, wie Acetonitril, Methylenchlorid, niedrige aliphatische Alkohole mit 1 bis 5 Kohlenstoffatomen, Dimethylformamid usw., innerhalb einer sehr kurzen Zeit statt. Die Umsetzung verläuft unter Ausschuss von atmosphärischer Feuchtigkeit.

Durch rasche Umsetzung bei Zimmer- oder leicht erhöhter Temperatur wird die gewünschte Verbindung mit einer hohen Ausbeute, sogar über 90 %, erhalten.

Bis-/2-(4-isobutylphenyl)-propionsäure/-anhydrid kann in quantitativer Ausbeute nach bekannten Methoden auch so hergestellt

werden, dass man zwei Moleküle der 2-(4-Isobutylphenyl)-propionsäure in Anwesenheit von N,N'-Dicyclohexylcarbodiimid (DCC) bei Zimmer- oder leicht erhöhter Temperatur in Anwesenheit von inerten Lösungsmitteln, wie Chloroform, Methylenchlorid, Acetonitril usw., dehydriert.

Bei Verwendung von Methylenchlorid als Lösungsmittel ist die Isolierung von Bis[2-(4-isobutylphenyl)-propionsäure]-anhydrid nicht nötig und die Lösung des Anhydrids in Methylenchlorid kann nach Entfernung von N,N'-Dicyclohexylharnstoff direkt in der Synthese von Ibuproxam eingesetzt werden. In diesem Fall wird die gewünschte Substanz in hoher Reinheit und auf einfache Weise isoliert, so dass sich eine weitere Reinigung durch Umkristallisieren erübrigt.

Die Gesamtausbeute der Umsetzung von 2-(4-Isobutylphenyl)-propionsäure zu 2-(4-Isobutylphenyl)-propiohydroxamsäure beträgt bei der Verwendung von Methylenchlorid als inertem Lösungsmittel über 90 %.

Reaktionsschema:

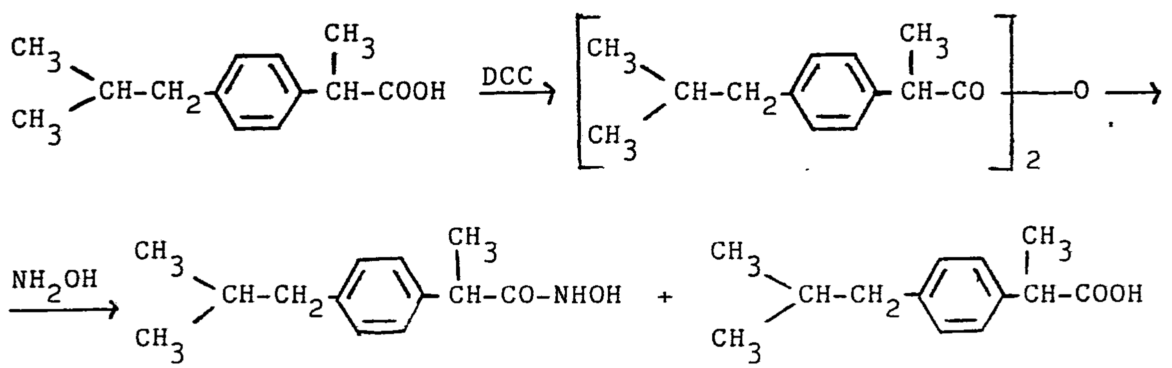


Die bei der Umsetzung entstandene 2-(4-Isobutylphenyl)-propionsäure wird erneut als Ausgangsstoff in das Verfahren zurückgeführt.

A)

Bis-[2-(4-isobutylphenyl)-propionsäure]-anhydrid

I.

2,3 g (0,011 Mol) 2-(4-Isobutylphenyl)-propionsäure werden in 15 ml Chloroform gelöst. Die Lösung wird mit 1,15 g (0,0055 Mol) N,N'-Dicyclohexylcarbodiimid (DCC) versetzt und das Reaktionsgemisch wird unter Ausschluss von Luftfeuchtigkeit 0,5 bis 1 Stunde bei Zimmertemperatur gerührt. Der ausgeschiedene N,N'-Dicyclohexylharnstoff wird abfiltriert. Das Lösungsmittel wird aus dem Filtrat abgedampft; es werden 2 g (90 %) eines öligen Produkts erhalten, welches als solches in der Synthese der gewünschten Verbindung eingesetzt werden kann.

II.

2,3 g (0,011 Mol) 2-(4-Isobutylphenyl)-propionsäure werden in 15 ml Methylenchlorid gelöst. Die Lösung wird mit 1,15 g (0,0055 Mol) N,N'-Dicyclohexylcarbodiimid (DCC) versetzt und das Reaktionsgemisch wird 0,5 bis 1 Stunde bei einer Temperatur von etwa 30°C und unter Ausschluss der Luftfeuchtigkeit gerührt. Der ausgeschiedene N,N'-Dicyclohexylharnstoff wird abgefiltert. Das Filtrat wird durch Eindampfen vom Lösungsmittel befreit; es werden 2,19 g (100%) eines öligen Produktes erhalten, welches als solches für die Synthese der gewünschten Verbindung eingesetzt werden kann.

$^1$H NMR (DMSO, 20°C)

$\tau_1$ = 9,07 [d, -CH-(C$\underline{H}_3$)$_2$]
$\tau_2$ = 8,55 [d, (CH$_3$)$_2$-C$\underline{H}$]
$\tau_3$ = 8,15 [m, CH$_2$C$\underline{H}$(CH$_3$)$_2$]
$\tau_4$ = 7,57 [d, C$\underline{H}_2$-CH]
$\tau_5$ = 6,3 [q, C$\underline{H}$-CH$_3$]
$\tau_6$ = 2,9 [s, Ph]

B)

2-(4-Isobutylphenyl)-propiohydroxamsäure

I.

2 g (0,005 Mol) Bis-/2-(4-isobutylphenyl)-propionsäure/-anhydrid in der Form des öligen Produktes werden in 10 ml Acetonitril gelöst und unter Ausschluss von Luftfeuchtigkeit mit 0,2 g (0,006 Mol) Hydroxylamin versetzt. Das Reaktionsgemisch wird bei Zimmertemperatur 2 Stunden gerührt. Das Gemisch wird danach eingedampft, der Rückstand wird in 50 ml Methylenchlorid gelöst und mit einer wässerigen Natriumhydroxyd-Lösung bei einem pH von etwa 8 extrahiert. Die organische Phase wird eingedampft und der Rückstand wird aus einem Petroläther/Aceton-Gemisch umkristallisiert. Es werden 0,9 g (80 %) 2-(4-Isobutylphenyl)-propiohydroxamsäure erhalten. Diese Verbindung entspricht gemäss den analytischen Daten der nach dem in der DE-PS 2 400 531 beschriebenen Verfahren hergestellten Bezugsverbindung (Ibuproxam)

Aus der wässerigen Phase wird 2-(4-Isobutylphenyl)-propionsäure isoliert, die wieder als Ausgangsstoff verwendet wird.

II.

2 g (0,005 Mol) Bis-/2-(4-isobutylphenyl)-propionsäure/-anhydri in Form des öligen Produkts werden in 10 ml Methylenchlorid gelöst und unter Ausschluss von Luftfeuchtigkeit mit 0,18 g (0,0055 Mol) Hydroxylamin versetzt. Das Reaktionsgemisch wird bei Raumtemperatur 1 Stunde gerührt. Danach wird das Gemisch eingedampft, der Rückstand wird mit 15 ml Petroläther versetzt und 1 bis 2 Stunden gerührt. Das ausgeschiedene Produkt wird abgefiltert und mit Petroläther nachgewaschen. Es werden 1,13 g (92 %) 2-(4-Isobutylphenyl)-propiohydroxamsäure, Smp. 119-121°C, erhalten.

Aus der Petroläther-Lösung wird 2-(4-Isobutylpehnyl)-propionsäure isoliert, die wieder als Ausgangsstoff verwendet wird.

III.

2 g (0,005 Mol) Bis-/2-(4-isobutylphenyl)-propionsäure/-anhydrid werden in 10 ml Acetonitril gelöst und mit 0,4 g (0,0057 Mol)

Hydroxylamin-Hydrochlorid versetzt. Die Lösung wird bei Raumtemperatur 1 Stunde gerührt, dann mit 10 ml einer 30 %igen Natriummethylat-Lösung in Methanol neutralisiert, eine weitere Stunde bei Raumtemperatur gerührt, eingedampft, und der Rückstand wird in 50 ml Methylenchlorid gelöst. Das ausgeschiedene NaCl wird abgefiltert und die Methylenchlorid-Phase wird mit einer wässrigen NaOH-Lösung bei einem pH von etwa 8 extrahiert. Die organische Phase wird eingedampft und der Rückstand wird aus einem Petroläther/Aceton-Gemisch umkristallisiert. Es werden 0,75 g 2-(4-Isobutylphenyl)-propiohydroxamsäure erhalten.

IV.

2 g (0,005 Mol) Bis /2-(4-isobutylphenyl)-propionsäure/-anhydrid werden in 10 ml Methylenchlorid gelöst und mit 0,4 g (0,0057 Mol) Hydroxylamin-Hydrochlorid versetzt. Die Lösung wird 1 Stunde bei Raumtemperatur gerührt und dann mit 10 ml einer 30 %igen Natriummethylat-Lösung in Methanol neutralisiert. Das Reaktionsgemisch wird eine weitere Stunde bei Raumtemperatur gerührt, das ausgeschiedene NaCl abgefiltert, der Rückstand wird mit 15 ml Petroläther versetzt und dann noch 1 bis 2 Stunden gerührt. Das ausgeschiedene Produkt wird abgefiltert und mit Petroläther nachgewaschen. Es werden 0,86 g 2-(4-Isobutylphenyl)-propiohydroxamsäure erhalten.

C)

<u>2-(4-Isobutylphenyl)-propiohydroxamsäure (ohne vorherige Isolierung von intermediärem Anhydrid)</u>

2,3 g (0,011 Mol) 2-(4-Isobutylphenyl)-propionsäure werden in 15 ml Methylenchlorid gelöst. Die Lösung wird mit 1,15 g (0,0055 Mol) N,N'-Dicyclohexylcarbodiimid (DCC) versetzt und das Reaktionsgemisch wird 0,5 bis 1 Stunde bei einer Temperatur von etwa 30°C unter Ausschluss von Luftfeuchtigkeit gerührt. Der ausgeschiedene N,N'-Dicyclohexylharnstoff wird abgefiltert. Das Filtrat wird unter Ausschluss von Luftfeuchtigkeit mit 0,18 g (0,0055 Mol) Hydroxylamin versetzt. Das Reaktionsgemisch wird bei Raumtemperatur etwa 1 Stunde gerührt. Das Gemisch wird dann

eingedampft, der Rückstand wird mit Petroläther versetzt und noch 1 bis 2 Stunden weitergerührt. Das ausgeschiedene Produkt wird abgefiltert und mit Petroläther nachgewaschen. Es werden 1,13 g 2-(4-Isobutylphenyl)-propiohydroxamsäure erhalten, die hinsichtlich der analytischen Ergebnisse dem authentischen Muster entspricht.

Aus der Petroläther-Lösung werden 1,1 g 2-(4-Isobutylphenyl)-propionsäure isoliert, die wieder als Ausgangsstoff eingesetzt wird.

PATENTANSPRUECHE L 1259 EU

1. Verfahren zur Herstellung von 2-(4-Isobutylphenyl)-propiohydroxamsäure der Formel

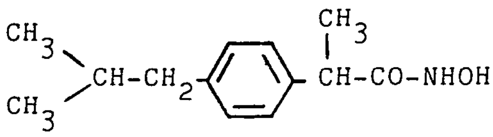

dadurch gekennzeichnet, dass man Bis-[2-(4-isobutylphenyl)-propionsäure]-anhydrid der Formel

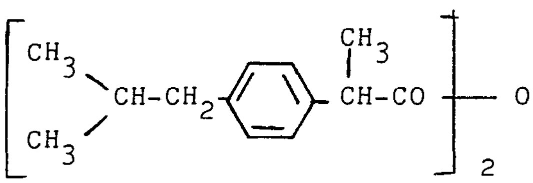

mit Hydroxylamin bzw. seinem Hydrochloridsalz in einem inerten organischen Lösungsmittel unter Ausschluss von Luftfeuchtigkeit bei einer Temperatur zwischen 0 und 60°C umsetzt.

2. Verfahren nach Anspruch 1, dadurch gekennzeichnet, dass man als inertes organisches Lösungsmittel Acetonitril, Methylenchlorid oder Chloroform verwendet.

Europäisches Patentamt

# EUROPÄISCHER RECHERCHENBERICHT

0203379

Nummer der Anmeldung

EP 86 10 5676

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | DE-A-2 658 610 (HISAMITSU PHARMACEUTICAL CO. INC.) * Seite 9, Arbeitsweise IV; Seite 29, Zeilen 12,13 * --- | 1,2 | C 07 C 83/10 |
| A | GB-A- 894 119 (IMPERIAL CHEMICAL INDUSTRIES) * Spalte 1, Zeilen 16-29 * --- | 1,2 | |
| A,D | METHODEN DER ORGANISCHEN CHEMIE, 4. Auflage, Band VIII, 1952,Thieme Verlag, Stuttgart, Seite 687, Zeilen 40-41 ----- | 1 | |
| | | | RECHERCHIERTE SACHGEBIETE (Int. Cl.4) |
| | | | C 07 C 83/00 |

Der vorliegende Recherchenbericht wurde für alle Patentansprüche erstellt.

| Recherchenort | Abschlußdatum der Recherche | Prüfer |
|---|---|---|
| BERLIN | 04-08-1986 | PROBERT C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument